# EUROPEAN PATENT APPLICATION

(11) **EP 4 834 909 A1**
(43) Date of publication of application: **04.11.2026**
(21) Application number: 24911594.0
(22) Date of filing: 29.12.2024
(51) Int. Cl.: C07D 498/04, C07D 519/00, A61K 31/5383, A61P 35/00, A61P 37/00

(54) **CRYSTAL FORM OF COMPOUND CONTAINING TRICYCLIC HETEROARYL, AND PREPARATION METHOD THEREFOR AND USE THEREOF**

(30) Priority: 29.12.2023 CN 202311850017
(71) Applicant: CSPC Zhongqi Pharmaceutical Technology (Shijiazhuang) Co., Ltd., Shijiazhuang, Hebei 050035 (CN); CSPC OUYI Pharmaceutical co., Ltd., Shijiazhuang, Hebei 052160 (CN); Hangzhou Innogate Pharma Co., Ltd., Hangzhou, Zhejiang 311121 (CN)
(72) Inventor: WANG, Jie, Shijiazhuang, Hebei 050035 (CN); GONG, Denghuang, Shijiazhuang, Hebei 050035 (CN); YAN, Cuiqin, Shijiazhuang, Hebei 050035 (CN); GE, Jinghua, Shijiazhuang, Hebei 050035 (CN); ZHANG, Hancheng, Hangzhou, Zhejiang 311121 (CN); YANG, Min, Shijiazhuang, Hebei 050035 (CN); MA, Yuxiu, Shijiazhuang, Hebei 050035 (CN); ZHU, Lifeng, Shijiazhuang, Hebei 050035 (CN)
(74) Representative: Barrow, Nicholas Martin
(86) International application number: PCT/CN2024/143563
(87) International publication number: WO 2025/140680

(57) **Abstract**

Provided in the present disclosure are a crystal form I of compound I, and a preparation method therefor and the use thereof. The crystal form I provided in the present disclosure has low hygroscopicity, good stability and micromeritic properties, is suitable for industrial production, and is suitable for being used as a raw material agent for production, storage and use.

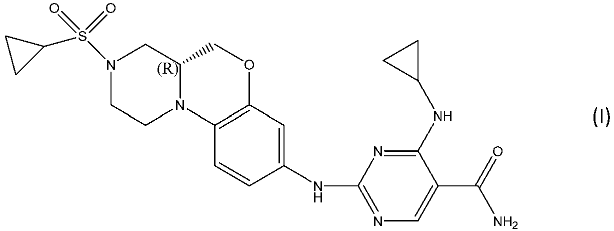

## Description

### TECHNICAL FIELD

The present disclosure belongs to the field of pharmaceutical chemistry, and particularly relates to a crystal form of a compound containing tricyclic heteroaryl, and a preparation method therefor and the use thereof.

### BACKGROUND ART

Janus kinase (JAK) is a non-transmembrane, non-receptor tyrosine kinase and comprises four subtypes: JAK1, JAK2, JAK3 and TYK2 (Tyrosine kinase 2). JAK inhibitors specifically inhibit the JAK-STAT (signal transducers and activators of transcription) signaling pathway, thereby blocking the cascade amplification of the above cytokines and participating in processes such as immune regulation.

Spleen tyrosine kinase (SYK) is a non-receptor tyrosine kinase found in the cytoplasm. SYK is widely expressed in hematopoietic cells, lymphocytes, fibroblasts, and vascular endothelial cells, is highly expressed in B lymphocytes, and plays an important role in tumors and autoimmune diseases. The SYK gene can inhibit the proliferation and migration of malignant tumor cells, such as breast cancer cells, melanoma cells, and liver cancer cells. Currently, SYK inhibitors have been used in clinical phase II/III trials for rheumatoid arthritis, chronic lymphocytic leukemia, and other conditions. Recent studies have shown that using SYK inhibitors or interfering with the SYK gene expression can effectively slow the progression of liver fibrosis/cirrhosis, demonstrating good therapeutic effects (see CN 105664178 A).

Compound I is a highly selective dual-target inhibitor of JAK kinase and SYK kinase, as disclosed in CN 110734454 A, with the structural formula as shown in formula I below:

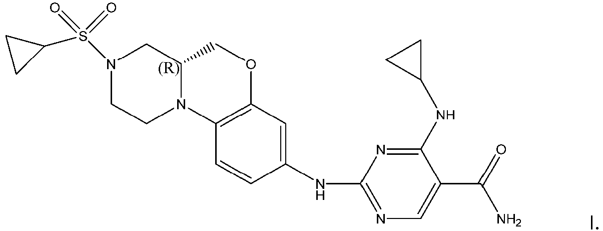

There is currently no research on the crystal form of compound I in the prior art.

### SUMMARY OF THE INVENTION

After extensive experimental research on the crystal form of compound I, the inventors finally developed a crystal form with low hygroscopicity, good stability (under conditions of high temperature, high humidity, and light exposure) and micromeritic properties, which is suitable for industrial production and is suitable for being used as a raw material agent for production, storage and use.

In a first aspect, the present disclosure provides a crystal form I of compound I, wherein as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 16.0 ± 0.2°, and 21.6 ± 0.2°,

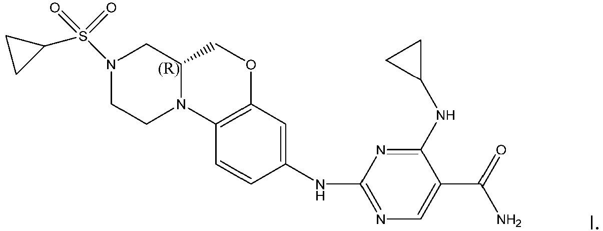

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 16.0 ± 0.2°, 19.2 ± 0.2°, and 21.6 ± 0.2°.

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 13.8 ± 0.2°, 16.0 ± 0.2°, 19.2 ± 0.2°, and 21.6 ± 0.2°.

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 13.8 ± 0.2°, 16.0 ± 0.2°, 18.7 ± 0.2°, 19.2 ± 0.2°, and 21.6 ± 0.2°.

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 13.8 ± 0.2°, 16.0 ± 0.2°, 18.7 ± 0.2°, 19.2 ± 0.2°, 20.6 ± 0.2°, and 21.6 ± 0.2°.

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 13.2 ± 0.2°, 13.8 ± 0.2°, 16.0 ± 0.2°, 18.7 ± 0.2°, 19.2 ± 0.2°, 20.6 ± 0.2°, and 21.6 ± 0.2°.

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 13.2 ± 0.2°, 13.8 ± 0.2°, 16.0 ± 0.2°, 18.7 ± 0.2°, 19.2 ± 0.2°, 20.6 ± 0.2°, 21.6 ± 0.2°, and 23.0 ± 0.2°.

In some embodiments of the present disclosure, the crystal form I is characterized in that as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 13.2 ± 0.2°, 13.8 ± 0.2°, 16.0 ± 0.2°, 18.7 ± 0.2°, 19.2 ± 0.2°, 20.6 ± 0.2°, 21.6 ± 0.2°, 23.0 ± 0.2°, and 25.0 ± 0.2°.

In the present disclosure, in the X-ray powder diffraction pattern (determined using Cu-Kα radiation and expressed in 2θ (°)) of the crystal form I, a doublet appears at 13.2 ± 0.2°, wherein the difference between the peak positions of the two peaks is between 0.1° and 0.4°, for example between 0.2° and 0.3°, and the intensity of the peaks is greater than 10%, for example greater than 15% or greater than 20%.

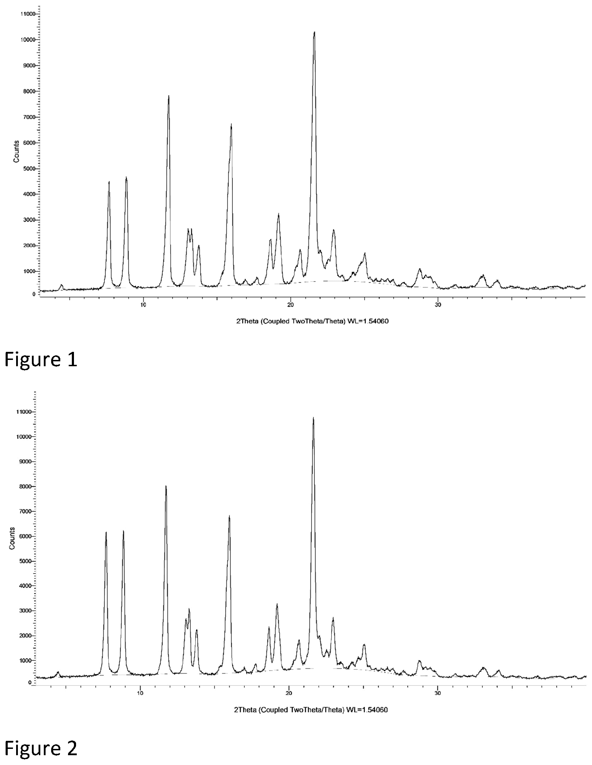

In some embodiments of the present disclosure, the crystal form I, as determined by using Cu-Kα radiation, has an X-ray powder diffraction pattern substantially as shown in FIG. 1 or FIG. 2.

In a second aspect, the present disclosure provides a method for preparing the above crystal form I, which method comprises: dissolving crude compound I in a dissolution solvent (solvent 1, or a mixed solvent of solvent 1/solvent 2); optionally adding seed crystals and solvent 3; performing crystallization; optionally subjecting the resulting solid to slurrying with optionally added solvent 4; performing separation to obtain the crystal form I, wherein the solvent 2 is absent, and the solvent 1 is acetone or acetonitrile; or the dissolution solvent is selected from one of the following mixed solvents (solvent 1/solvent 2): acetone/ethyl acetate, trifluoroethanol/water, and ethanol/dichloromethane, wherein when the solvent 2 is water, the water accounts for no more than 10% by volume of the total volume of solvent 1 and solvent 2; the solvent 3 is n-heptane; the solvent 4 is selected from acetone, ethyl acetate, acetone-isopropyl acetate (preferably in a volume ratio of 3 : 1), acetone-methanol (preferably in a volume ratio of 1 : 3), and ethanol.

In some embodiments of the present disclosure, in the above preparation method, the volume-to-mass ratio (mL/g) of the solvent 1 to the crude compound I is (5-500) : 1, preferably (5-350) : 1, more preferably (5-200) : 1, further preferably (7-70) : 1, even further preferably (10-30) : 1, and the volume-to-mass ratio (mL/g) of the solvent 2 to the crude compound I is (0-200) : 1, more preferably (0-150) : 1, further preferably (0-50) : 1, even further preferably (15-50) : 1.

In some embodiments of the present disclosure, in the above preparation method, the volume ratio of the solvent 1 to the solvent 2 is (1-20) : (0-50), preferably (1-15) : (0-30), more preferably (1-15) : (0-10), further preferably (1-15) : (0-5), even further preferably (1-3) : (0-5).

In some embodiments of the present disclosure, in the above preparation method, the dissolution solvent is acetone, acetonitrile, acetone/ethyl acetate (preferably in a volume ratio of 5 : 1), trifluoroethanol/water (preferably in a volume ratio of 10 : 1), or ethanol/dichloromethane (preferably in a volume ratio of (1-3) : (1.5-5)); the solvent 3 is preferably n-heptane; the solvent 4 is acetone, ethyl acetate, acetone-isopropyl acetate (preferably in a volume ratio of 3 : 1), acetone-methanol (preferably in a volume ratio of 1 : 3), or ethanol.

In some embodiments of the present disclosure, in the above preparation method, the solvent 2 is absent, and the solvent 1 is the dissolution solvent acetone (preferably the crystallization temperature is 4°C) or acetonitrile (preferably the crystallization temperature is 4°C), or the dissolution solvent is selected from one of the following mixed solvents (solvent 1/solvent 2): acetone/ethyl acetate (preferably the volume ratio is 5 : 1, and/or the crystallization temperature is 4°C), trifluoroethanol/water (preferably the volume ratio is 10 : 1, and/or the crystallization temperature is 4°C), ethanol/dichloromethane (preferably the volume ratio is (1-3) : (1.5-5), and/or the crystallization temperature is room temperature); the solvent 3 is preferably n-heptane; the solvent 4 is acetone, ethyl acetate, acetone-isopropyl acetate (preferably in a volume ratio of 3 : 1), acetone-methanol (preferably in a volume ratio of 1 : 3), or ethanol.

In some embodiments of the present disclosure, in the above preparation method, the solvent 1 is preferably ethanol; the solvent 2 is preferably dichloromethane; the solvent 3 is preferably n-heptane; the solvent 4 is preferably acetone.

In some embodiments of the present disclosure, in the above preparation method, the dissolution step optionally comprises adding a base, wherein the base can be an inorganic base or an organic base, preferably an organic base, more preferably an organic amine, such as triethylamine.

In some embodiments of the present disclosure, in the above preparation method, preferably the method comprises adding seed crystals before crystallization.

In some embodiments of the present disclosure, in the above preparation method, the crystallization temperature is -50°C to 50°C, preferably 0°C to 45°C; more preferably room temperature or 4°C.

In some embodiments of the present disclosure, in the above preparation method, the slurrying is performed at room temperature.

In some embodiments of the present disclosure, in the above preparation method, the separation step comprises using a suitable method such as filtration and centrifugation to separate the obtained crystal form I from the crystallization mother liquor.

In some embodiments of the present disclosure, in the above preparation method, in order to remove free solvent from the product, the preparation method comprises a drying step after the separation step. The drying method can be any suitable known method, preferably drying under reduced pressure (in vacuum). Specific drying conditions comprise, for example, a temperature preferably of 30°C-70°C, further preferably of 35°C-60°C, more preferably of 40°C-50°C; and a drying time preferably of 4-20 h, more preferably of 8-16 h. Regardless of the drying method used, the content of residual solvent in the resulting product should ideally meet the quality standards.

The crude compound I in the present disclosure can be prepared using the method disclosed in CN 110734454 A, or any other known methods disclosed in the prior art. Specifically, the "crude compound I" herein refers to a solid or an equivalent thereof (in terms of purity) that can be prepared according to the method in Example 29 of CN 110734454 A.

In a third aspect, the present disclosure also provides a pharmaceutical composition comprising the above crystal form I and optionally a pharmaceutically acceptable carrier.

The above pharmaceutical composition is formulated into clinically acceptable preparations, for example, oral preparations, injectable preparations, topical administration preparations, and external preparations, preferably oral preparations. The oral preparation is preferably a solid preparation, such as a tablet, a capsule, and a granule. The preparations can be prepared using corresponding excipients known to those of ordinary skill in the art by corresponding known techniques for preparing pharmaceutical preparations.

In a fourth aspect, the present disclosure also provides the use of the above crystal form I or the above pharmaceutical composition:
(a) in the manufacture of a medicament for treating a disease associated with protein kinase activity or expression level;
(b) in the manufacture of a protein kinase-targeted inhibitor; and/or
(c) for in vitro non-therapeutic inhibition of protein kinase activity;
wherein the protein kinase is selected from, but not limited to, the group consisting of: SYK, JAK, or a combination thereof.

In some embodiments of the present disclosure, the disease associated with protein kinase activity or expression level is selected from: autoimmune disease, hematologic malignancy, and solid tumor;
preferably, the disease associated with protein kinase activity or expression level is selected from: breast cancer, melanoma, rheumatoid arthritis, chronic lymphocytic leukemia, monocytic leukemia, splenomegaly with erythrocytosis, eosinophilic leukocytosis syndrome, primary thrombocytopenia, systemic giant cell diseases, liver cancer, rectal cancer, bladder cancer, throat cancer, non-small cell lung cancer, small cell lung cancer, lung adenocarcinoma, lung squamous cell carcinoma, prostate cancer, glioma, ovarian cancer, head and neck squamous cell carcinoma, cervical cancer, esophageal cancer, kidney cancer, pancreatic cancer, colon cancer, skin cancer, lymphoma, gastric cancer, multiple myeloma, liver fibrosis/cirrhosis, allergic asthma, myelofibrosis, B-cell lymphoma, psoriasis, atopic dermatitis, lupus erythematosus, and diseases associated with cytokine release syndrome and systemic inflammatory response syndrome.

Preferably, the psoriasis is selected from: psoriasis vulgaris, pustular psoriasis, erythrodermic psoriasis, and psoriatic arthritis.

Preferably, the lupus erythematosus is selected from: discoid lupus erythematosus, subacute cutaneous lupus erythematosus, systemic lupus erythematosus, lupus erythematosus profundus, neonatal lupus erythematosus and drug-induced lupus erythematosus.

Preferably, the diseases associated with cytokine release syndrome or systemic inflammatory response syndrome are selected from: (1) cytokine release syndrome or systemic inflammatory response syndrome caused by infection, (2) cytokine release syndrome or systemic inflammatory response syndrome caused by non-infectious factors such as trauma, burns, surgery, or ischemia-reperfusion injury, (3) cytokine release syndrome or systemic inflammatory response syndrome caused by hemorrhagic shock, ischemia, tissue injury, multiple trauma, acute pancreatitis, burns, poisoning, or drug-induced fever, and (4) cytokine release syndrome or systemic inflammatory response syndrome caused by immunotherapy; the infection is caused by bacteria, viruses, fungi, etc., such as respiratory tract infection, biliary tract infection, intra-abdominal infection and traumatic infection; the virus is preferably: coronavirus, influenza virus, Ebola virus, hepatitis C virus, Dengue virus, etc.; the coronavirus is preferably: SARS virus, novel coronavirus (COVID-19), and MERS virus; the influenza virus is preferably: influenza A virus and influenza B virus; preferably, the diseases associated with cytokine release syndrome or systemic inflammatory response syndrome are: pneumonia, COVID-19 or acute lung injury requiring oxygen supplementation, pneumonia, COVID-19 or acute lung injury requiring non-invasive or invasive mechanical ventilation, or hospitalized pneumonia, COVID-19 or acute lung injury requiring extracorporeal membrane oxygenation; the immunotherapy is CART therapy.

The "patient" described above includes all members of the animal kingdom, including but not limited to mammals (such as mice, rats, cats, monkeys, and dogs) and humans.

### Definition and Description

Unless otherwise stated, the following terms and phrases used herein are intended to have the following meanings. A specific phrase or term should not be considered uncertain or unclear unless specifically defined, but should be understood in an ordinary meaning. When a trade name appears herein, it is intended to refer to the corresponding commercial product or an active ingredient thereof.

Unless otherwise specified, the "2θ, 2θ angle or angle 2θ" described in the present disclosure refers to a diffraction angle in ° or degrees.

Unless otherwise specified, with regard to the "crystallization temperature" and "drying temperature" described in the present disclosure, the unit is °C (degree Celsius), and the margin of error can be ± 10°C, ± 5°C, ± 4°C, ± 3°C, ± 2°C, or ± 1°C.

The term "substantially as shown in the figure" refers to that at least 50%, or at least 60%, or at least 70%, or at least 80%, or at least 90%, or at least 95%, or at least 96%, or at least 97%, or at least 98%, or at least 99% of the peaks in the X-ray powder diffraction pattern, or DSC pattern, or crystal morphology diagram of the crystalline particles of a substantially pure specific crystal form appear in the provided pattern. Further, when the content of a specific crystal form within a product gradually decreases, some diffraction peaks in the X-ray powder diffraction pattern thereof that are attributable to that specific crystal form may decrease in number due to the detection sensitivity of the instrument. Additionally, it is also well known in the field of crystallography that slight errors in peak positions may exist for any given crystal form. For example, peak positions may shift due to factors such as temperature fluctuations during sample analysis, sample displacement, or instrument calibration, and the measurement error of the 2θ value is typically about ± 0.2°. Therefore, when determining the structure of each crystal form, this error should be taken into account. The term "substantially" or "substantially as shown in the figure" is also intended to encompass such variations in diffraction peak positions.

Herein, room temperature refers to 10°C-30°C, preferably 20°C-30°C.

The present disclosure is described in detail by the following examples.

The solvents used in the present disclosure are commercially available.

### Technical Effects

The crystal form obtained in the present disclosure has the following beneficial effects:
(1) The crystal form of the present disclosure has low hygroscopicity.
(2) The crystal form of the present disclosure has excellent stability. On the one hand, the crystal form of the present disclosure has excellent physical stability, and remains unchanged after air jet milling; on the other hand, the crystal form of the present disclosure has excellent thermodynamic stability. When the crystal form of the present disclosure is placed under high temperature, high humidity, and light exposure conditions, the content of the tested sample shows no significant change and the crystal form remains unchanged, making it more suitable for being used as a raw material agent for storage and use.
(3) The crystal form of the present disclosure has excellent micromeritic properties, making it suitable for the preparation of a pharmaceutical preparation, particularly an oral pharmaceutical preparation.

### BRIEF DESCRIPTION OF THE DRAWINGS

FIG. 1: an XRPD pattern of crystal form I of compound I obtained in Example 1.
FIG. 2: an XRPD pattern of crystal form I of compound I obtained in Example 3.
FIG. 3: an XRPD pattern of crystal form II of compound I obtained in Example 5.
FIG. 4: an XRPD pattern of crystal form I after air jet milling.

### DETAILED DESCRIPTION OF EMBODIMENTS

### X-ray powder diffractometer (XRPD)

Instrument model (Examples 5-7 and Comparative Example): Bruker D8 Advance X-ray diffractometer
Target: Cu (40 KV, 40 mA)
Step size: 0.02°
Scanning range: 3°-40° 2θ
Scanning speed: 0.02°/0.2 s; or
Instrument model (Examples 1-4 and Test Examples 3-4): Bruker D2 PHASER X-ray diffractometer
Target: Cu (30 KV, 10 mA)
Step size: 0.02°
Scanning range: 3°-40° 2θ
Scanning speed: 0.02°/0.3 s.

Preferred examples of the present disclosure will be described in detail below with reference to the accompanying drawings. The examples provided are intended to better illustrate the content of the present disclosure, but the content of the present disclosure is not limited to the provided examples. Those skilled in the art can make non-essential improvements and adjustments to the embodiments based on the above content of the present disclosure, which still fall within the scope of protection of the present disclosure.

### Preparative Example: Preparation of crude compound I

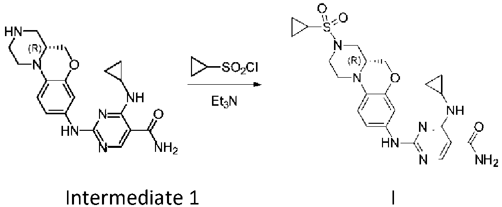

The crude compound I, a pale yellow solid, was prepared with reference to Example 29 of CN 110734454 A. The resulting solid sample was subjected to X-ray powder diffraction testing, and the result indicated an amorphous form.

### Example 1: Preparation of crystal form I of compound I

3 g of crude compound I was added to a reaction flask, and 500 mL of acetone and 100 mL of ethyl acetate were added. The mixture was heated to 50°C for dissolution, and stirred at the same temperature for 10 min for crystallization, and then the resulting mixture was cooled to 4°C and stirred for 3 h for crystallization to obtain a white crystal (2.55 g, HPLC purity: 99.87%). X-ray powder diffraction testing of the sample was performed. The results reveal that the sample is crystal form I, the corresponding pattern is shown in FIG. 1, and the data results are shown in Table 1. TGA testing of the sample was performed. The results reveal that the sample is an anhydrous crystal form.

**Table 1: X-ray powder diffraction peaks of crystal form I sample from Example 1**

| **2θ (°)** | **Relative intensity (%)** | **2θ (°)** | **Relative intensity (%)** |
|---|---|---|---|
| 7.663 | 42.2 | 18.654 | 17.6 |
| 8.843 | 45.3 | 19.178 | 29.0 |
| 11.716 | 77.7 | 20.647 | 13.6 |
| 13.051 | 33.9 | 21.610 | 100 |
| 13.265 | 34.4 | 22.928 | 20.8 |
| 13.789 | 16.3 | 25.022 | 12.0 |
| 15.966 | 66.4 | | |

### Example 2: Preparation of crystal form I of compound I

The crystal form was prepared with reference to the preparation method of Example 1, with the crystallization solvent changed. The results are as follows:

| Example | Solvent 1 | Solvent 2 | Result |
|---|---|---|---|
| 2-1 | 500 mL of acetone | -- | Crystal form I |
| 2-2 | 1000 mL of acetonitrile | -- | Crystal form I |
| 2-3 | 200 mL of trifluoroethanol | 20 mL of water | Crystal form I |

### Example 3: Preparation of crystal form I of compound I

10 g of crude compound I was added to a reaction flask, and 100 mL of ethanol, 200 mL of dichloromethane, and 3 mL of triethylamine were added. The mixture was stirred at room temperature for dissolution, the crystal form I sample obtained in Example 1 was added as seed crystals, and 249 mL of n-heptane was added. The resulting mixture was stirred at room temperature for crystallization to obtain a white crystal (about 8.1 g). The wet white crystal was subjected to slurrying in acetone to obtain crystal form **I** (HPLC purity: 99.98%). X-ray powder diffraction testing of the sample was performed. The results reveal that the sample is crystal form I, the corresponding pattern is shown in FIG. 2, and the data results are shown in Table 2.

**Table 2: X-ray powder diffraction peaks of crystal form I sample from Example 3**

| **2θ (°)** | **Relative intensity (%)** | **2θ (°)** | **Relative intensity (%)** |
|---|---|---|---|
| 7.708 | 57.2 | 18.661 | 17.4 |
| 8.884 | 57.5 | 19.203 | 26.5 |
| 11.732 | 75.1 | 20.684 | 11.6 |
| 13.103 | 23.0 | 21.647 | 100.0 |
| 13.312 | 24.8 | 22.939 | 18.7 |
| 13.735 | 15.2 | 25.027 | 9.9 |
| 15.990 | 63.1 | | |

The investigation shows that crystal form I can be obtained with ethanol at 10-30 v/w : dichloromethane at 15-50 v/w (note: v/w refers to the volume-to-mass ratio of solvent to crude product, mL/g).

### Example 4: Investigation of preparation conditions for crystal form I of compound I

The crystal form was prepared with reference to the preparation method of Example 3, with the slurrying solvent changed. The results of the resulting crystal form are as follows:

| Example | Slurry solvent | Result |
|---|---|---|
| 5-1 | 660 mL of ethyl acetate | Crystal form I |
| 5-2 | 300 mL of acetone and 100 mL of isopropyl acetate | Crystal form I |
| 5-3 | 100 mL of acetone and 300 mL of methanol | Crystal form I |
| 5-4 | 100 mL of ethanol | Crystal form I |

### Example 5: Preparation of crystal form II of compound I

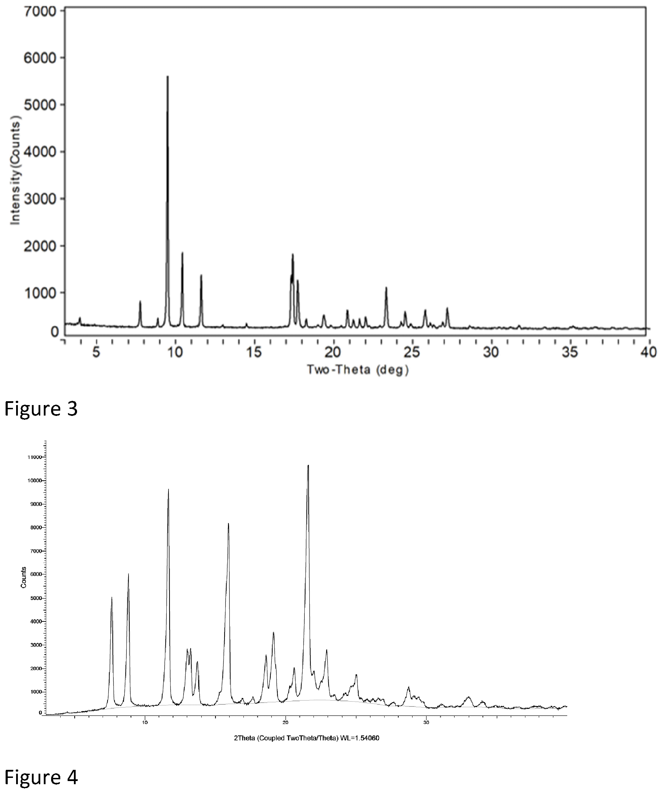

About 300 mg of the sample from Preparative Example 1 was added to 30 mL of acetonitrile and 10 mL of water, and the mixture was heated to give a clear solution, filtered, and evaporated to dryness in an open vessel at 40°C to obtain a solid (290 mg, yield: 96.7%, HPLC purity: 99.96%). X-ray powder diffraction testing of the sample was performed. The results reveal that the sample is crystal form II, the corresponding pattern is shown in FIG. 3, and the data results are shown in Table 3. TGA testing of the sample was performed. The results reveal that the sample is a dihydrate crystal form.

**Table 3: X-ray powder characteristic peaks of crystal form II from Example 5**

| **2θ (°)** | **Relative intensity (%)** | **2θ (°)** | **Relative intensity (%)** |
|---|---|---|---|
| 7.762 | 11 | 21.238 | 3.5 |
| 9.495 | 100 | 21.637 | 3.6 |
| 10.430 | 31.8 | 22.004 | 6.3 |
| 11.612 | 23.2 | 23.331 | 22.7 |
| 17.408 | 52.6 | 24.515 | 9.8 |
| 17.733 | 25.3 | 25.807 | 11.1 |
| 19.388 | 7.9 | 27.201 | 12.2 |
| 20.873 | 8.7 | | |

Crystal form II can also be obtained by replacing the solvent with tetrahydrofuran/water (2.0/1.0) and evaporating to dryness at room temperature.

### Example 6: Preparation of crystal form II of compound I

30 mg of crude compound I was added to a reaction flask, and solvent 1 and solvent 2 (as below) were added. The mixture was heated to 50°C for dissolution, and then the resulting mixture was cooled to 4°C and stirred for crystallization to obtain crystal form II.

| Example | Solvent 1 | Solvent 2 | Result |
|---|---|---|---|
| 7-1 | 1.0 mL of tetrahydrofuran | 0.2 mL of water | Crystal form II |
| 7-2 | 1.5 mL of acetone | 0.6 mL of water | Crystal form II |
| 7-3 | 6.0 mL of acetonitrile | 1.6 mL of water | Crystal form II |

### Example 7: Preparation of crystal form II of compound I

With reference to the preparation of crystal form I of compound I, crystal form II was obtained under the following conditions.

| Example | Temperature (°C) | Solvent 1 | Solvent 2 | Result |
|---|---|---|---|---|
| 8-1 | Room temperature | 0.8 mL of acetone | 1.0 mL of water | Crystal form II |
| 8-2 | Room temperature | 0.2 mL of 1,4-dioxane | 1.8 mL of water | Crystal form II |
| 8-3 | 40 | 0.4 mL of acetonitrile | 1.6 mL of water | Crystal form II |

### Test Example 1: Solubility test of crystal form

Appropriate amounts of the amorphous sample from the Preparative Example, the crystal form I sample from Example 3, and the crystal form II sample from Example 5 were weighed and subjected to solubility tests in water, respectively. The results showed that the solubilities of the three substances described above fell within the same solubility class as defined in the Pharmacopoeia of the People's Republic of China (2020 Edition, Volume II).

### Test Example 2: Hygroscopicity test of crystal form

Appropriate amounts of the amorphous sample from the Preparative Example, the crystal form I sample from Example 3, and the crystal form II sample from Example 5 were weighed. In accordance with the Guidelines for the Testing of Hygroscopicity of Drugs, the samples were placed in a suitable constant-temperature desiccator (with a saturated solution of ammonium chloride or ammonium sulfate at the bottom) at 25°C ± 1°C for 24 h to perform the hygroscopicity test. The results showed that: the average weight gain of the crystal form I sample was 0.8%, indicating slight hygroscopicity; the average weight gain of the crystal form II sample was 47.9%, indicating significant hygroscopicity; the average weight gain of the amorphous sample was 3.0%, indicating hygroscopicity.

### Test Example 3: Stability test of crystal form:

Appropriate amounts of the amorphous sample from the Preparative Example, the crystal form I sample from Example 3, and the crystal form II sample from Example 5 were weighed and placed under conditions of high temperature (60°C), high humidity (25°C/75% RH) and light exposure (4500 ± 500 lux) for 5 and 10 days, respectively. The resulting data were compared with those at day 0. The experimental results are shown in Table 4.

**Table 4: Stability test results for amorphous form, crystal form I, and crystal form II**

| **Condition** | **Time/day** | **Amorphous form** | | | |
|---|---|---|---|---|---|
| | | **Appearance** | **Maximum single impurity (%)** | **Total impurity (%)** | **Crystal form** |
| -- | 0 | Pale yellow solid | 1.58 | 6.91 | - |
| High temperature (60°C) | 5 | Pale yellow solid | 1.54 | 6.92 | Unchanged |
| | 10 | Pale yellow solid | 3.17 | 9.71 | Unchanged |
| High humidity (25°C/75% RH) | 5 | Pale yellow solid | 1.51 | 7.03 | Unchanged |
| | 10 | Pale yellow solid | 1.46 | 7.85 | Unchanged |
| Light exposure (4500 ± 500 lux) | 5 | Pale yellow solid | 1.45 | 7.16 | Unchanged |
| | 10 | Pale yellow solid | 1.63 | 8.00 | Unchanged |

| **Condition** | **Time/day** | **Crystal form I** | | | |
|---|---|---|---|---|---|
| | | **Appearance** | **Maximum single impurity (%)** | **Total impurity (%)** | **Crystal form** |
| -- | 0 | Off-white solid | 0.01 | 0.02 | - |
| High temperature (60°C) | 5 | Off-white solid | 0.02 | 0.05 | Unchanged |
| | 10 | Off-white solid | 0.02 | 0.05 | Unchanged |
| High humidity (25°C/75% RH) | 5 | Off-white solid | 0.02 | 0.04 | Unchanged |
| | 10 | Off-white solid | 0.02 | 0.05 | Unchanged |
| Light exposure (4500 ± 500 lux) | 5 | Off-white solid | 0.02 | 0.05 | Unchanged |
| | 10 | Off-white solid | 0.02 | 0.06 | Unchanged |

| **Condition** | **Time/day** | **Crystal form II** | | | |
|---|---|---|---|---|---|
| | | **Appearance** | **Maximum single impurity (%)** | **Total impurity (%)** | **Crystal form** |
| -- | 0 | Off-white solid | 0.03 | 0.04 | Unchanged |
| High temperature (60°C) | 5 | Off-white solid | 0.04 | 0.06 | Transformed |
| | 10 | Off-white solid | 0.03 | 0.04 | Transformed |
| High humidity (25°C/75% RH) | 5 | Off-white solid | 0.04 | 0.06 | Unchanged |
| | 10 | Off-white solid | 0.04 | 0.05 | Unchanged |
| Light exposure (4500 ± 500 lux) | 5 | Off-white solid | 0.04 | 0.05 | Transformed |
| | 10 | Off-white solid | 0.04 | 0.05 | Transformed |

The results showed that after the crystal form I was placed under conditions of high temperature (60°C), high humidity (25°C/75% RH) and light exposure (4500 ± 500 lux) for 5 and 10 days, respectively, there were essentially no changes in appearance, related substances, or crystal form; after the crystal form II was placed under conditions of high temperature (60°C), high humidity (25°C/75% RH) and light exposure (4500 ± 500 lux) for 5 and 10 days, respectively, there were essentially no changes in appearance and related substances; however, the crystal form underwent a transformation under conditions of high-temperature and light exposure (note: XRPD detection revealed the presence of characteristic peaks other than those of crystal form II); the amorphous form underwent degradation under conditions of high temperature, high humidity, and light exposure, with the most significant degradation occurring under condition of high temperature.

### Test Example 4: Determination of milling stability of crystal form I

An appropriate amount of the crystal form I sample from Example 3 was pulverized using a laboratory-scale jet mill (feed gas pressure: 6-7 bar, pulverizing gas pressure: 6-7 bar). The pulverized sample was subjected to XRPD detection, and no change in crystal form was observed. The corresponding pattern is shown in FIG. 4, and the data results are shown in Table 5.

**Table 5: X-ray powder diffraction peaks of crystal form I after air jet milling**

| **2θ (°)** | **Relative intensity (%)** | **2θ (°)** | **Relative intensity (%)** |
|---|---|---|---|
| 7.642 | 47.2 | 18.604 | 20.6 |
| 8.831 | 57.0 | 19.134 | 30.1 |
| 11.678 | 93.2 | 20.528 | 11.2 |
| 13.018 | 35.6 | 21.610 | 100.0 |
| 13.274 | 36.1 | 22.919 | 22.8 |
| 13.739 | 18.6 | 24.877 | 8.2 |
| 15.932 | 78.4 | | |

### Test Example 5: Micromeritic property testing

The micromeritic properties of the crystal form I sample from Example 3 were tested. The test results are as follows:

| Test item | Test result |
|---|---|
| Angle of repose | 38° |
| Bulk density | 0.272 g/ml |
| Tap density | 0.354 g/ml |
| Hausner ratio | 1.30 |

### Comparative Example:

During research and development, the inventors found that compound I could not form crystalline solids via various crystallization methods and experimental conditions; only oil, solution, emulsion or amorphous form was obtained. Alternatively, only mixed crystals could be obtained under various experimental conditions. Exemplary solutions include, but are not limited to, the following comparative examples:

### Comparative Example 1

About 30 mg of the sample from Preparative Example 1 was weighed and added to 12 mL of trifluoroethanol. The mixture was stirred for dissolution, and the resulting solution was concentrated to dryness under reduced pressure to obtain an oil.

### Comparative Example 2: Anti-solvent crystallization method

Forward addition: About 30 mg of the sample from Preparative Example 1 was weighed and dissolved in solvent 1 at 40°C to give a clear solution, and the solution was filtered. Solvent 2 was added dropwise to the solvent 1, and the mixture was stirred at room temperature for crystallization.

Reverse addition: About 50 mg of the sample from Preparative Example 1 was weighed and dissolved in solvent 1 at 40°C to give a clear solution, and the solution was filtered. The solvent 1 was added dropwise to solvent 2, and the mixture was stirred at room temperature for crystallization.

| No. | Method | Solvent 1 | Solvent 2 | Solvent 1/solvent 2 (mL/mL) | Result |
|---|---|---|---|---|---|
| 2-1 | Forward addition | Butanone | Butyl acetate | 8.0/12.0 | Solution |
| 2-2 | Forward addition | Dimethyl sulfoxide | Ethyl acetate | 0.2/5.0 | Solution |
| 2-3 | Forward addition | Dimethyl sulfoxide | Isopropyl ether | 0.2/5.0 | Emulsion |
| 2-4 | Reverse addition | Dimethyl sulfoxide | Dichloromethane | 0.2/5.0 | Solution |
| 2-5 | Forward addition | Trifluoroethanol | Isopropyl acetate | 7.0/13.0 | Amorphous form |

### Comparative Example 3: Cooling crystallization method

About 30 mg of the sample from Preparative Example 1 was weighed, and a solvent was added. Then, the mixture was warmed to 50°C to give a clear solution, and the solution was filtered and stirred at 4°C for crystallization.

| No. | Solvent 1 | Solvent 2 | Solvent 1/solvent 2 (mL/mL) | Result |
|---|---|---|---|---|
| 3-1 | Trifluoroethanol | -- | 5.0 | Amorphous form |
| 3-2 | Trifluoroethanol | Butyl acetate | 5.0/3.0 | Amorphous form |
| 3-3 | Dimethyl sulfoxide | Ethyl acetate | 0.3/0.3 | Solution |
| 3-4 | Dimethyl sulfoxide | Chloroform | 0.3/0.4 | Solution |

### Comparative Example 4: Slurry crystallization method

About 30 mg of the sample from Preparative Example 1 was weighed, a solvent was added, and the mixture was placed at the corresponding temperature for crystallization for 5 days.

| No. | Temperature | Solvent 1 | Solvent 2 | Solvent 1/solvent 2 (mL/mL) | Result |
|---|---|---|---|---|---|
| 4-1 | Room temperature | 1,4-Dioxane | -- | 2.0 | Solution |
| 4-2 | Room temperature | Butanone | 1,4-Dioxane | 1.0/1.0 | Solution |
| 4-3 | Room temperature | Dimethyl sulfoxide | Isopropyl ether | 0.1/1 | Emulsion |
| 4-4 | Room temperature | Methanol | Dichloromethane | 1/4 | Mixed crystal |

### Comparative Example 5: Evaporation crystallization method

About 30 mg of the sample from Preparative Example 1 was weighed, the corresponding solvent was added, and the mixture was ultrasonically dissolved and evaporated to dryness in an open vessel at the corresponding temperature.

| No. | Temperature | Solvent 1 | Solvent 2 | Solvent 1/solvent 2 (mL/mL) | Result |
|---|---|---|---|---|---|
| 5-1 | 40 | Trifluoroethanol | Water | 5.0/1.0 | Amorphous form |
| 5-2 | 40 | Trifluoroethanol | Acetone | 11.0/3.0 | Amorphous form |
| 5-3 | Room temperature | Trifluoroethanol | Isopropyl ether | 7.0/1.0 | Amorphous form |
| 5-4 | 40 | Trifluoroethanol | Ethyl acetate | 7.0/3.0 | Amorphous form |
| 5-5 | 40 | Trifluoroethanol | Dichloromethane | 7.0/5.0 | Amorphous form |
| 5-6 | 40 | Acetone | Water | 6.0/1.0 | Mixed crystal |

## Claims

1. A crystal form I of compound I, wherein as determined using Cu-Kα radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 16.0 ± 0.2°, and 21.6 ± 0.2°,

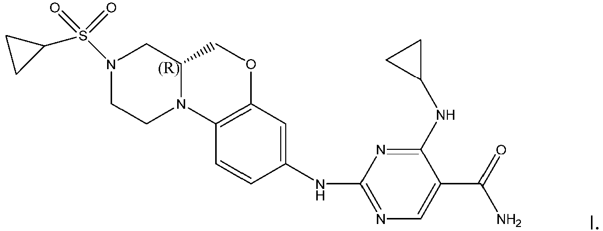

2. The crystal form I of compound I according to claim 1, wherein as determined using Cu-Ka radiation and expressed in 2θ (°), the crystal form I has an X-ray powder diffraction pattern comprising characteristic diffraction peaks at 7.7 ± 0.2°, 8.9 ± 0.2°, 11.7 ± 0.2°, 16.0 ± 0.2°, 19.2 ± 0.2°, and 21.6 ± 0.2°;
or the crystal form I has an X-ray powder diffraction pattern comprising characteristic diffraction peaks at 7.7 ± 0.2°, 8.9 ± 0.2°, 11.7 ± 0.2°, 13.8 ± 0.2°, 16.0 ± 0.2°, 19.2 ± 0.2°, and 21.6 ± 0.2°;
or the crystal form I has an X-ray powder diffraction pattern comprising characteristic diffraction peaks at 7.7 ± 0.2°, 8.9 ± 0.2°, 11.7 ± 0.2°, 13.8 ± 0.2°, 16.0 ± 0.2°, 18.7 ± 0.2°, 19.2 ± 0.2°, and 21.6 ± 0.2°;
or the crystal form I has an X-ray powder diffraction pattern comprising characteristic diffraction peaks at 7.7 ± 0.2°, 8.9 ± 0.2°, 11.7 ± 0.2°, 13.8 ± 0.2°, 16.0 ± 0.2°, 18.7 ± 0.2°, 19.2 ± 0.2°, 20.6 ± 0.2°, and 21.6 ± 0.2°;
or the crystal form I has an X-ray powder diffraction pattern comprising characteristic diffraction peaks at 7.7 ± 0.2°, 8.9 ± 0.2°, 11.7 ± 0.2°, 13.2 ± 0.2°, 13.8 ± 0.2°, 16.0 ± 0.2°, 18.7 ± 0.2°, 19.2 ± 0.2°, 20.6 ± 0.2°, and 21.6 ± 0.2°;
or the crystal form I has an X-ray powder diffraction pattern comprising characteristic diffraction peaks at 7.7 ± 0.2°, 8.9 ± 0.2°, 11.7 ± 0.2°, 13.2 ± 0.2°, 13.8 ± 0.2°, 16.0 ± 0.2°, 18.7 ± 0.2°, 19.2 ± 0.2°, 20.6 ± 0.2°, 21.6 ± 0.2°, and 23.0 ± 0.2°;
or the crystal form I has an X-ray powder diffraction pattern comprising characteristic diffraction peaks at 7.7 ± 0.2°, 8.9 ± 0.2°, 11.7 ± 0.2°, 13.2 ± 0.2°, 13.8 ± 0.2°, 16.0 ± 0.2°, 18.7 ± 0.2°, 19.2 ± 0.2°, 20.6 ± 0.2°, 21.6 ± 0.2°, 23.0 ± 0.2°, and 25.0 ± 0.2°;
or the crystal form I has an X-ray powder diffraction pattern substantially as shown in FIG. 1 or FIG. 2.

3. A pharmaceutical composition, comprising the crystal form I of compound I according to claim 1 or 2 and optionally a pharmaceutically acceptable carrier.

4. The pharmaceutical composition according to claim 3, wherein the pharmaceutical composition is formulated into clinically acceptable preparations, including oral preparations, injectable preparations, topical administration preparations, external preparations, etc.; preferably oral preparations, and further preferably tablets, capsules, and granules.

5. Use of the crystal form I of compound I according to claim 1 or 2 or the pharmaceutical composition according to claim 3 or 4 in the manufacture of a medicament for treating diseases (a), (b), and/or (c):
(a) in the manufacture of a medicament for treating a disease associated with protein kinase activity or expression level;
(b) in the manufacture of a protein kinase-targeted inhibitor; and/or
(c) for in vitro non-therapeutic inhibition of protein kinase activity;
preferably, the protein kinase is selected from, but not limited to, the group consisting of: SYK, JAK, or a combination thereof.

6. The use according to claim 5, wherein the disease associated with protein kinase activity or expression level is selected from: autoimmune disease, hematologic malignancy, and solid tumor;
preferably, the disease associated with protein kinase activity or expression level is selected from: breast cancer, melanoma, rheumatoid arthritis, chronic lymphocytic leukemia, monocytic leukemia, splenomegaly with erythrocytosis, eosinophilic leukocytosis syndrome, primary thrombocytopenia, systemic giant cell diseases, liver cancer, rectal cancer, bladder cancer, throat cancer, non-small cell lung cancer, small cell lung cancer, lung adenocarcinoma, lung squamous cell carcinoma, prostate cancer, glioma, ovarian cancer, head and neck squamous cell carcinoma, cervical cancer, esophageal cancer, kidney cancer, pancreatic cancer, colon cancer, skin cancer, lymphoma, gastric cancer, multiple myeloma, liver fibrosis/cirrhosis, allergic asthma, myelofibrosis, B-cell lymphoma, psoriasis, atopic dermatitis, lupus erythematosus, and diseases associated with cytokine release syndrome or systemic inflammatory response syndrome.

7. A method for preparing the crystal form I of compound I according to claim 1 or 2, comprising:
dissolving crude compound I in solvent 1 and solvent 2; optionally adding seed crystals and solvent 3; performing crystallization; optionally subjecting the resulting solid to slurrying with optionally added solvent 4; performing separation to obtain the crystal form I,
wherein
the solvent 2 is absent, and the solvent 1 is acetone or acetonitrile; or a mixed solvent of the solvent 1/solvent 2 is selected from one of the following mixed solvents: acetone/ethyl acetate, trifluoroethanol/water, and ethanol/dichloromethane, wherein when the solvent 2 is water, the water accounts for no more than 10% by volume of the total volume of solvent 1 and solvent 2; the solvent 3 is n-heptane;
the solvent 4 is selected from acetone, ethyl acetate, acetone-isopropyl acetate (preferably in a volume ratio of 3 : 1), acetone-methanol (preferably in a volume ratio of 1 : 3), or ethanol.

8. The preparation method according to claim 7, wherein the volume-to-mass ratio (mL/g) of the solvent 1 to the crude compound I is (5-500) : 1, preferably (5-350) : 1, more preferably (5-200) : 1, further preferably (7-70) : 1, even further preferably (10-30) : 1, and the volume-to-mass ratio (mL/g) of the solvent 2 to the crude compound I is (0-200) : 1, more preferably (0-150) : 1, further preferably (0-50) : 1, even further preferably (15-50) : 1.

9. The preparation method according to claim 7, wherein the volume ratio of the solvent 1 to the solvent 2 is (1-20) : (0-50), preferably (1-15) : (0-30), more preferably (1-15) : (0-10), further preferably (1-15) : (0-5), even further preferably (1-3) : (0-5).

10. The preparation method according to claim 7, wherein the solvent 1 is ethanol; the solvent 2 is dichloromethane; the solvent 3 is n-heptane; the solvent 4 is acetone.

11. The preparation method according to claim 7, wherein the dissolution step optionally comprises adding a base, wherein the base can be an inorganic base or an organic base, preferably an organic base, more preferably an organic amine, such as triethylamine.

12. The preparation method according to claim 7, wherein the method comprises adding seed crystals before crystallization.

13. The preparation method according to claim 7, wherein the crystallization temperature is -50°C to 50°C, preferably 0°C to 45°C; more preferably room temperature or 4°C.
